# EUROPEAN PATENT SPECIFICATION

(11) **EP 4 153 185 B1**
(45) Date of publication and mention of the grant of the patent: **02.12.2026**
(21) Application number: 21725556.1
(22) Date of filing: 19.05.2021
(51) Int. Cl.: A61K 31/702, A23L 33/00, A23L 33/125, A61K 45/06, A61P 19/08

(54) **USE OF HUMAN MILK OLIGOSACCHARIDES IN NUTRITIONAL COMPOSITIONS FOR ENHANCING BONE DEVELOPMENT AND/OR BONE STRENGTH**
VERWENDUNG VON MUTTERMILCHOLIGOSACCHARIDEN IN NÄHRSTOFFZUSAMMENSETZUNGEN ZUR VERBESSERUNG DER KNOCHENENTWICKLUNG UND/ODER KNOCHENSTÄRKE
UTILISATION D'OLIGOSACCHARIDES DU LAIT HUMAIN DANS DES COMPOSITIONS NUTRITIONNELLES POUR AMÉLIORER LE DÉVELOPPEMENT OSSEUX ET/OU LA RÉSISTANCE OSSEUSE

(30) Priority: 22.05.2020 EP 20175969
(43) Date of publication of application: 29.03.2023
(73) Proprietor: Société des Produits Nestlé S.A., 1800 Vevey (CH)
(72) Inventor: HORCAJADA, Marie, Noëlle, 01170 ECHENEVEX (FR); BONNET, Nicolas, 74380 NANGY (FR); RAMOS, NIEVES, José, Manuel, 1004 LAUSANNE (CH); BARUCHET, Michaël, Frédéric, Henri, 1807 BLONAY (CH)
(74) Representative: Elkington and Fife LLP
(86) International application number: PCT/EP2021/063220
(87) International publication number: WO 2021/233960

(56) References cited:
- WO-A1-2013/057063
- JP-A- 2008 169 168
- CARTER S A ET AL: "Infant milk feeding and bone health in later life: findings from the Hertfordshire cohort study", OSTEOPOROSIS INTERNATIONAL, SPRINGER LONDON, LONDON, vol. 31, no. 4, 15 February 2020 (2020-02-15), pages 709 - 714, XP037063983, ISSN: 0937-941X, [retrieved on 20200215], DOI: 10.1007/S00198-020-05296-1
- JONES G ET AL: "Breastfeeding in early life and bone mass in prepubertal children: A longitudinal study", vol. 11, no. 2, 1 February 2000 (2000-02-01), London, pages 146 - 152, XP093267337, ISSN: 0937-941X, Retrieved from the Internet <URL:https://link.springer.com/article/10.1007/PL00004176> DOI: 10.1007/PL00004176
- ANDRES ALINE ET AL: "Body Fat and Bone Mineral Content of Infants Fed Breast Milk, Cow's Milk Formula, or Soy Formula during the First Year of Life", vol. 163, no. 1, 1 July 2013 (2013-07-01), US, pages 49 - 54, XP093267338, ISSN: 0022-3476, Retrieved from the Internet <URL:https://www.sciencedirect.com/science/article/pii/S0022347612015569?via%3Dihub> DOI: 10.1016/j.jpeds.2012.12.067
- SPRENGER NORBERT ET AL: "Longitudinal change of selected human milk oligosaccharides and association to infants' growth, an observatory, single center, longitudinal cohort study", PLOS ONE, 9 February 2017 (2017-02-09), pages 1 - 15, XP093267319, Retrieved from the Internet <URL:https://pmc.ncbi.nlm.nih.gov/articles/PMC5300226/pdf/pone.0171814.pdf> DOI: 10.1371/journal.pone.0171814February9,2017
- CHARBONNEAU MARK R ET AL: "Sialylated Milk Oligosaccharides Promote Microbiota-Dependent Growth in Models of Infant Undernutrition", CELL, ELSEVIER, AMSTERDAM NL, vol. 164, no. 5, 18 February 2016 (2016-02-18), pages 859 - 871, XP029433856, ISSN: 0092-8674, DOI: 10.1016/J.CELL.2016.01.024

## Description

### Technical Field

The present invention relates to a synthetic nutritional composition comprising a mixture of oligosaccharides, said mixture containing at least one sialylated oligosaccharide and at least one fucosylated oligosaccharide, for use in enhancing bone strength in a subject, wherein the at least one sialylated oligosaccharide is a combination of 3'-sialyllactose (3'-SL) and 6'-sialyllactose (6'-SL), the at least one fucosylated oligosaccharide is a combination of 2'-fucosyllactose (2'FL) and 2',3-difucosyllactose (LDFT), the composition further comprises at least one N-acetylated oligosaccharide which is a combination of lacto-N-tetraose (LNT) and lacto-N-neotetraose (LNnT), and wherein enhancing bone strength decreases cortical porosity (CtPo).

### Background of the Invention

Bone growth during infancy is a key parameter with respect to bone strength during human life.

Ossification, or osteogenesis, is the process of bone formation by osteoblasts. Ossification is distinct from the process of calcification: whereas calcification takes place during the ossification of bones, it can also occur in other tissues.

Ossification (formation of new bone) begins approximately six weeks after fertilization in an embryo. Before this time, the embryonic skeleton consists entirely of fibrous membranes and hyaline cartilage. Bone growth continues until approximately age 25. Bones can grow in thickness throughout life, but after age 25, ossification functions primarily in bone remodeling and repair.

Bone is a dynamic tissue which undergoes remodeling, namely a life-long process consisting of resorption (the breaking down of old bone) and ossification, and is key to shaping the skeleton and to repairing bone fractures.

Bone is composed of cortical (or compact) bone and trabecular (or spongy) bone. Cortical bone accounts for approximately 80% of the mass of bone of the human body, and has a lower surface area than trabecular bone due to its lower porosity.

Trabecular bone is located at the end of long bones and accounts for approximately 20% of the total mass of the skeleton.

Bone strength is the joint result of the action of several parameters which are involved in bone growth. In this respect, not only factors such as age, gender, location in the body, mineral content, disease etc. have an impact on bone strength, but also the trabecular architecture ( form and orientation of trabeculae) and the cortical microarchitecture (in particular, as regards the repartition of porosity) play a significant role in bone health and strength.

A composition for use in the promotion of healthy bone growth and/or in the prevention and/or treatment of bone disease, comprising at least one probiotic mixture and a mixture of oligosaccharides, said mixture containing at least one N-acetylated oligosaccharide, at least one sialylated oligosaccharide and at least one neutral oligosaccharide, is disclosed in WO2013/057063.

WO2018/135719 relates to a composition containing sialyllactose for preventing or treating osteoarthritis by inhibiting cartilage destruction or promoting cartilage formation.

The article by C.A. Cowardin et al., PNAS, 2019, 116 reports the study of interactions among the gut microbiota, human milk oligosaccahrides (HMOs) and osteoclast and osteoblast biology, in order to identify breast milk components that influence postnatal. This article states that the HMO 2'-fucosyllalctose(2'-FL) failed to elicit changes in bone-biology.

The article by M.R. Charbonneau et al., Cell, 2016, 164, 859-871 reviews the role of sialylated milk oligosaccharides for promoting microbiota-dependent growth in infants with undernutrition.

Breast feeding is considered as the ideal source of nutrition and is the preferred choice for feeding infants up to at least 6 months of age. Consequently, human milk (HM) has long been considered as the model for the design of infant formulas (IF). Even many improvements in the nutrient composition of IF have been made during the last decades, there are still important differences in composition as well as in functional benefits conveyed by HM.

As a consequence, regarding bone metabolism, a lower bone mineral density can, for example, be observed in formula fed infants compared to breastfed ones.

Therefore, there is a need for providing specific nutrients found in breastmilk to infant formulae in order to promote bone growth in formula fed infants or to obtain these beneficial effects in infants who do not get sufficient amounts of these nutrients through breastfeeding.

In particular, there is a need to provide a composition which can be used as an infant formula for promoting bone health and/or bone density and/or bone architecture.

### Summary of the Invention

It has now been found that a synthetic nutritional composition comprising a mixture of oligosaccharides containing at least one sialylated oligosaccharide and at least one fucosylated oligosaccharide, can be used for enhancing bone strength in a subject, wherein the at least one sialylated oligosaccharide is a combination of 3'-sialyllactose (3'-SL) and 6'-sialyllactose (6'-SL), the at least one fucosylated oligosaccharide is a combination of 2'-fucosyllactose (2'FL) and 2',3-difucosyllactose (LOFT), the composition further comprises at least one N-acetylated oligosaccharide which is a combination of lacto-N-tetraose (LNT) and lacto-N-neotetraose (LNnT), and wherein enhancing bone strength decreases cortical porosity (CtPo).

In particular, it was surprisingly found that the synthetic nutritional composition could be used for decreasing cortical porosity, and in embodiments, for increasing bone mineral density (BMD) and/or bone mineral content (BMC).

Advantageously, it was found that it could be extrapolated from the experimental studies that the beneficial effect on bone strength could be achieved as a long-lasting effect, namely that, for example with respect to bone mineral density (BMD) and/or bone mineral content (BMC), said beneficial effect remained at a later age, even though the administration of the composition had stopped.

Sialylated and fucosylated oligosaccharides are compounds found in human breast milk (human milk oligosaccharides). Accordingly, it may be particularly beneficial if the synthetic nutritional composition is administered to an infant or child, and in particular to an infant or child fed infant formula or growing up milk. Whilst breast-feeding is recommended for all infants, in some cases breast-feeding is insufficient or not possible for medical reasons. In these situations infant formula or growing up milks are a lifeline as they can be used as an alternative to mother's milk.

Accordingly, in an embodiment, the subject is a human infant or young child, and in a more specific embodiment still the subject is a human infant or child fed infant formula or growing up milk.

The invention further relates to a composition for use in enhancing bone strength as described above, wherein the oligosaccharide mixture comprises:
- 10 to 35 wt%, preferably 10 to 30 wt%, more preferably 10 to 25 wt%, with respect to the total weight of the oligosaccharide mixture, of at least one sialylated oligosaccharide;
- 30 to 80 wt%, preferably 40 to 80 wt%, more preferably 50 to 70 wt%, with respect to the total weight of the oligosaccharide mixture, of at least one fucosylated oligosaccharide, and,
- 10 to 35 wt%, preferably 15 to 30 wt%, more preferably 15 to 20 wt%, with respect to the total weight of the oligosaccharide mixture, of at least one N-acetylated oligosaccharide.

In a preferred embodiment of the invention, there is provided a composition, as described above, for increasing bone mineral density (BMD) and/or bone mineral content (BMC) and/or Bone volume and tissue volume fraction (BV/TV) and/or bone ultimate force (FMax) and/or elastic energy.

The composition of the invention can be in the form of an infant formula.

In this case, said infant formula can be a preterm infant formula, a human milk fortifier, a starter infant formula, a follow-on formula, a baby-food formula, an infant cereal formula, a growing-up milk, a medical food product for clinical nutrition or a supplement, typically to be used during hospital stay and/or after hospital discharge. A supplement can be for a preterm infant or a child or an adult. Said composition is preferably a product for preterm feeding such as a preterm infant formula, a human milk fortifier, or a supplement. The composition according to the invention can also be products for children or adults such as yoghurt or medical food, as well as pet's food, especially young pets, and specifically for the same benefits than those demonstrated by the data shown in the present description.

The composition according to the invention can be for use before and/or during and/or after a weaning period.

The invention further provides the use of the synthetic nutritional composition for enhancing bone strength in a human subject or a pet.

### Brief Description of the Figures

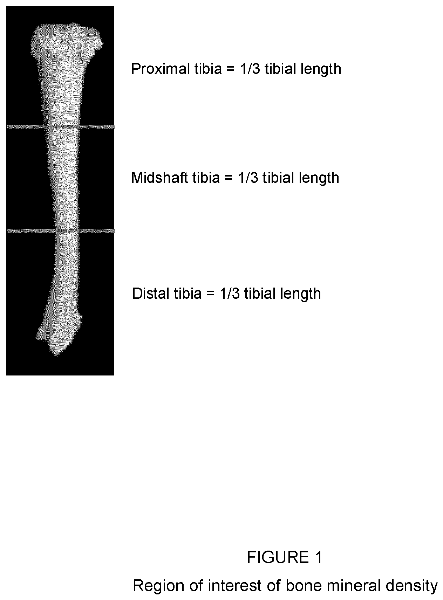

Figure 1 shows the region of interest of bone mineral density.

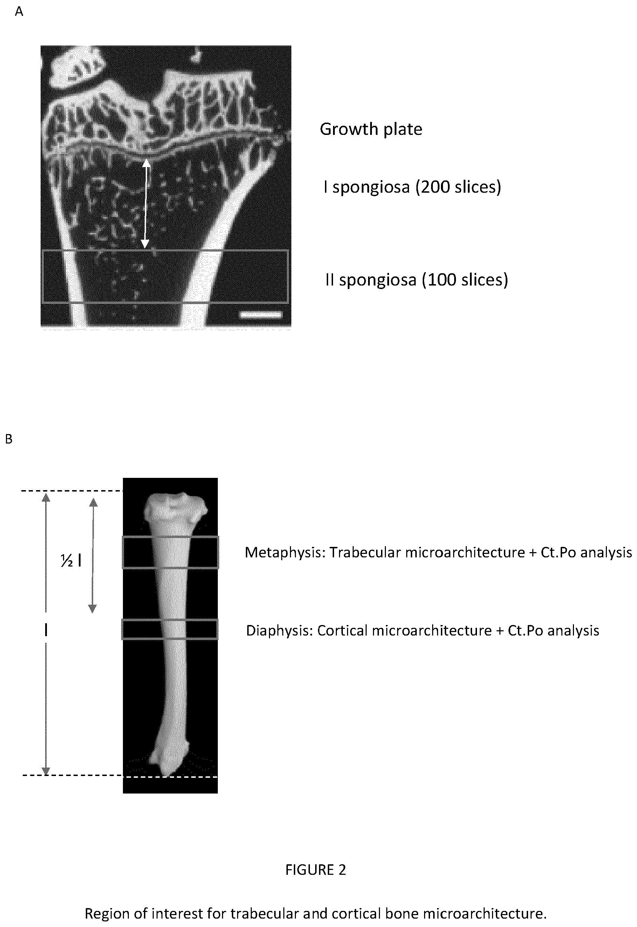

Figure 2 shows the region of interest for the measurement of trabecular (A) and cortical (B) microarchitecture.
Figure 3 shows the improvement in bone mineral density in minipiglets artificially reared with compositions according to the invention versus a lactose-fed group and a naturally-reared reference group.
Figure 4 shows the improvement in trabecular bone volume in minipiglets artificially reared with compositions according to the invention versus a naturally-reared reference group.
Figure 5 shows the prevention of increase of cortical porosity in minipiglets artificially reared with compositions according to the invention versus a lactose-fed group and a naturally-reared reference group.
Figure 6 shows the increase of bone ultimate force (FMax) and elastic energy in minipiglets artificially reared with compositions according to the invention versus a lactose-fed group and a naturally-reared reference group.

### Detailed Description

In a first aspect of the invention, there is provided a synthetic nutritional composition comprising a mixture of oligosaccharides, said mixture containing at least one sialylated oligosaccharide and at least one fucosylated oligosaccharide, for use in enhancing bone strength in a subject, wherein the at least one sialylated oligosaccharide is a combination of 3'-sialyllactose (3'-SL) and 6'-sialyllactose (6'-SL), the at least one fucosylated oligosaccharide is a combination of 2'-fucosyllactose (2'FL) and 2',3-difucosyllactose (LOFT), the composition further comprises at least one N-acetylated oligosaccharide which is a combination of lacto-N-tetraose (LNT) and lacto-N-neotetraose (LNnT), and wherein enhancing bone strength decreases cortical porosity (CtPo).

Within the context of the present invention, the term "enhancing bone strength" means, in particular, one or more of the following physiological processes: bone mass acquisition, optimization of peak bone mass, promotion of bone formation, promotion of bone anabolism, increase of bone mineral density and micro-architecture, modulation of bone biomechanical properties, modulation the ratio of bone formation and/or bone resorption, assist bone regeneration during fracture healing, regulation of bone resorption process.

Non-limiting example of sialylated oligosaccharides include: 3'-sialyllactose (3'-SL), 6'-sialyllactose (6'-SL). 3'-sialyllactose (3'-SL) and 6'-sialyllactose (6'-SL) are human milk oligosaccharides.

As used herein the term "3'-sialyllactose" (3'-SL, 3-SL, 3'SL, or 3SL), refers to (6R)-5-Acetamido-3,5-dideoxy-6-[(1R,2R)-1,2,3-trihydroxypropyl]-β-L-threo-hex-2-ulopyranonosyl-(2->3)-β-D-galactopyranosyl-(1->4)-D-glucopyranose (IUPAC).

As used herein the term "6'-sialyllactose" (6'-SL, 6-SL, 6'SL, or 6SL) refers to (6R)-5-Acetamido-3,5-dideoxy-6-[(1R,2R)-1,2,3-trihydroxypropyl]-β-L-threo-hex-2-ulopyranonosyl-(2->6)-β-D-galactopyranosyl-(1->4)-D-glucopyranose (IUPAC).

3'-sialyllactose (3'-SL), and/or 6'-sialyllactose (6'-SL) may be isolated by chromatographic or filtration technology from a natural source such as animal milks. Alternatively, they may be produced by biotechnological means using specific sialyltransferases or sialidases, neuraminidases, either by an enzyme based fermentation technology (recombinant or natural enzymes), by chemical synthesis or by a microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP), from DP=1 onwards. Alternatively, sialyllactoses may be produced by chemical synthesis from lactose and free N'-acetylneuraminic acid (sialic acid). Sialyllactoses are also commercially available for example from Kyowa Hakko Kogyo, Japan, or from GeneChem, Republic of Korea.

Fucosylated oligosaccharide(s) described herein include: 2'-fucosyllactose (2'FL), 3-fucosyllactose (3FL), 2',3-difucosyllactose (LOFT), difucosyllactose (diFL), lacto-N-fucopentaose , such as lacto-N-fucopentaose I (LNFP-I), lacto-N-fucopentaose II (LNFP-II), lacto-N-fucopentaose III (LNFP-III) or lacto-N-fucopentaose V (LNFP-V), lacto-N-fucohexaose, lacto-N-difucohexaose I, lacto-neofucopentaose V (LNnFP-V), lacto-N-difucosylhexaose-I (LNDFH-1), lacto-N-neodifucosylhexaose (LNnDFH), fucosyllacto-N-hexaose, fucosyllacto-N-neohexaose (such as fucosyllacto-N-neohexaose I, fucosyllacto-N-neohexaose II), monofucosyllacto-n-hexaose-III (MFNLH-III), difucosyllacto-N-hexaose I, difuco-lacto-N-neohexaose, difucosyllacto-N-neohexaose I, difucosyllacto-N-neohexaose II, difucosyllacto-N-hexaose-a (DFLNHa), fucosyl-para-Lacto-N-hexaose, tri-fuco-para-Lacto-N-hexaose I and combination thereof. In the claimed invention, 2'-fucosyllactose (2'FL) and 2',3-difucosyllactose (LDFT) are present in combination; the other listed fucosylated oligosaccharides may additionally be present.

In the claimed invention, the composition further comprises at least one N-acetylated oligosaccharide, which is a combination of lacto-N-tetraose (LNT) and lacto-N-neotetraose (LNnT).

Additional N-acetylated oligosaccharides that may be present in the composition include N-acetyl-glucosamine, N-acetyl-galactosamines and combinations thereof.

Further N-acetylated oligosaccharide(s) described herein include: LNT (lacto-N-tetraose), para-lacto-N-neohexaose (para-LNnH), LNnT (lacto-N-neotetraose) and any combinations thereof. Other examples are lacto-N-hexaose, lacto-N-neohexaose, para- lacto-N-hexaose, para-lacto-N-neohexaose, lacto-N-octaose, lacto-N- neooctaose, iso- lacto-N-octaose, para-lacto-N-octaose and lacto-N-decaose. In the claimed invention, LNT and LNnT are present in combination; the other listed N-acetylated oligosaccharides may additionally be present.

For an infant formula or growing up milk, the skilled person may base the amount of an HMO e.g. 2'FL, diFL, LNT, LNnT, 3SL and/or 6SL on the amounts found in human breast milk produced for an infant or child of the same age, in particular by a nutritionally replete mother. Such amounts may fall within the following ranges in human breast milk: diFL:100-500 mg/L, LNT: 50-300 mg/L, LNnT: 200-2000 mg/L, 2'FL : 500-3000 mg/L, 3'SL : 100-400 mg/L, 6'SL: 50-750 mg/L. However, they may be outside depending on for example bioavailability of said HMOs from infant formula in comparison to human breastmilk.

As a guide, for an infant formula or growing up milk, the fucosylated oligosaccharide(s) including 2'FL and 2',3-difucosyllactose (LOFT) in combination, may be present in the nutritional composition according to the present invention in a total amount of 0.1 to 4 g/L of the composition, for example in a total amount of 0.1 to 3.5 g/L of the composition for example 0.15 to 3 g/L, 0.2 to 2.5 g/L, 0.3 to 2 g/L, 0.4 to 2 g/L, 0.5 to 2 g/L of the composition (the concentration may refer to the concentration after the composition has been reconstituted e.g. with water).

In a particular embodiment, the composition comprises from 200 to 1800 mg of total fucosylated oligosaccharide(s) per L of the nutritional composition.

As a guide, for an infant formula or growing up milk, the sialylated oligosaccharide(s) including 3'-sialyllactose (3'-SL) and 6'-sialyllactose (6'-SL) in combination, may be present in the nutritional composition according to the invention in a concentration of from 50mg to 750mg/L for example from 50mg to 500mg/L for example from 100mg to 300mg per L, from 100mg to 400mg per L of the nutritional composition. In a particular embodiment, the composition comprises from 120mg to 400mg of total sialylated oligosaccharide(s) per L of the nutritional composition.

In the claimed composition, 3'-Sialyllactose (3'-SL) and 6'-Sialyllactose (6'-SL) may be present in a weight ratio between 10:1 and 1:10, such as between 10:1 and 2:1, between 8:1 and 3:1, between 6:1 and 3:1, between 5:1 and 3:1, between 5:1 and 4:1,or else between 1:2 to1.5:1

As a guide, for an infant formula or growing up milk, the N-acetylated oligosaccharide(s) including LNT and LNnT in combination may be present in the nutritional composition according to the present invention in a total amount of 0.05-to 0.5 g/L of the composition, for example in a total amount of 0.3 g/L of the composition, for example 0.35 g/L of the composition.

In a preferred embodiment, the composition comprises :
- 0.01 to 2wt%, preferably 0.05 to 1.5wt%, most preferably 0.07% to 1wt% of at least one sialylated oligosaccharide;
- 0.05 to 3wt%, preferably 0.1 to 2wt%, most preferably 0.2 to 1.5wt% of at least one fucosylated oligosaccharide, and,
- 0.01 to 1wt%, preferably 0.03 to 0.6wt%, most preferably 0.05 to 0.5wt% of at least one N-acetylated oligosaccharide,
with respect to the total weight of the infant formula under powder form.

The term "infant formula" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

Generally a "starter infant formula" is intended for infants from birth as breast-milk substitute.

A "follow-up formula" or "follow-on formula" is given from the 6th month onwards. It constitutes the principal liquid element in the progressively diversified diet of this category of person.

The term "preterm infant formula" as used herein means an infant formula intended for a preterm infant.

The term "milk fortifier" as used herein refers to liquid or solid nutritional compositions suitable for mixing with breast milk (which is human milk for a human milk fortifier) or infant formula. It is used to increase the calories, protein, minerals and vitamins in breast milk fed to preterm infants or infants with a low birth weight. The term "breast milk" is to be understood as the mother's milk or the colostrum of the mother or a donor's milk or the colostrum of a donor's milk.

The term "baby food formula" as used herein means a foodstuff intended for particular nutritional use by infants or children such as young children, during the first years of life.

The term "growing-up milk" (or GUM) as used herein refers to a milk formula product given from one year onwards. It is generally a diary -based beverage adapted for the specific nutritional needs of young children.

The term "infant cereal composition" as used herein refers to a foodstuff intended for particular nutritional use by infants or children such as young children, during the first years of life.

In another particular embodiment the nutritional composition of the present invention is a fortifier. The fortifier can be a breast milk fortifier or a formula fortifier such as an infant formula fortifier. The fortifier is therefore a particularly advantageous embodiment when the infant or young child is born preterm.

When the composition is a supplement, it can be provided in the form of unit doses.

The nutritional composition of the invention, and especially the infant formula, generally contains a protein source, a carbohydrate source and a lipid source. In some embodiments however, especially if the nutritional composition of the invention is a supplement or a fortifier, there may be only lipids (or a lipid source).

The nutritional composition according to the invention may contain a protein source. The protein may be in an amount of from 1.6 to 3 g per 100 kcal. In some embodiments, especially when the composition is intended for preterm infants/young children, the protein amount can be between 2.4 and 4 g/100kcal or more than 3.6 g/100kcal. In some other embodiments the protein amount can be below 2.0 g per 100 kcal, e.g. between 1.8 to 2 g/100kcal, or in an amount below 1.8g per 100 kcal.

Protein sources based on, for example, whey, casein and mixtures thereof may be used as well as plant based protein sources, for example, based on soy. As far as whey proteins are concerned, the protein source may be based on acid whey or sweet whey or mixtures thereof and may include alpha-lactalbumin and beta-lactoglobulin in any desired proportions. In some embodiments the protein source is whey predominant (i.e. more than 50% of proteins are coming from whey proteins, such as 60%> or 70%>). The proteins may be intact or hydrolysed or a mixture of intact and hydrolysed proteins. By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids.

The proteins may be either fully or partially hydrolysed. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10%> by weight of lysine; for example about 7% by weight of lysine which greatly improves the nutritional quality of the protein source.

In one particular embodiment the proteins of the composition are hydrolysed, fully hydrolysed or partially hydrolysed. The degree of hydrolysis (DH) of the protein can be between 2 and 20, or between 8 and 40, or between 20 and 60 or between 20 and 80 or more than 10, 20, 40, 60, 80 or 90. For example, nutritional compositions containing hydrolysates having a degree of hydrolysis less than about 15% are commercially available from Nestle Company under the trade mark Peptamen^{®}.

At least 70%, 80%, 85%, 90%, 95% or 97% of the proteins may be hydrolysed. In a particular embodiment, 100% of the proteins are hydrolysed.

In one particular embodiment the proteins of the composition are plant based protein.

The nutritional composition according to the present invention may contain a carbohydrate source. This is particularly preferable in the case where the nutritional composition of the invention is an infant formula. In this case, any carbohydrate source conventionally found in infant formulae such as lactose, sucrose, saccharose, maltodextrin, starch and mixtures thereof may be used although one of the preferred sources of carbohydrates for infant formula is lactose.

The nutritional composition according to the present invention may contain lipids and essential fatty acids.

Non limiting examples of lipids include: palm olein, high oleic sunflower oil, high oleic safflower oil, canola oil, fish oil, coconut oil, bovine milk fat, and combinations thereof. It may be particularly beneficial if the composition comprises fat in an amount of 25 to 30g/100g dry weight of the composition.

Non limiting examples of essential fatty acids include: linoleic acid (LA), α-linolenic acid (ALA). The compositions of the invention may further contain gangliosides monosialoganglioside-3 (GM3) and disialogangliosides 3 (GD3), and combinations thereof.

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B3, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population. If necessary, the nutritional composition of the invention may contain emulsifiers and stabilisers such as soy, lecithin, citric acid esters of mono- and diglycerides, and the like.

### Preparation of nutritional compositions:

The nutritional compositions according to the present invention may be prepared by any known or otherwise suitable manner. For example, an infant formula may be proposed by blending together a source of protein with a carbohydrate source and a lipid source in appropriate proportions. If used, emulsifiers may be included at this stage. Vitamins and minerals may be added at this stage, but may also be added later to avoid thermal degradation. Water, preferably water which has been subjected to reverse osmosis or deionized water, may then be added and mixed in to form a liquid mixture. The temperature of mixing is preferably room temperature, but may also be higher. The liquid mixture may then be thermally treated to reduce bacterial loads. The mixture may then be homogenized.

If it is desired to produce a powdered composition, the homogenized mixture is dried in a suitable drying apparatus, such as a spray drier or freeze drier and converted into powder.

Processes used in the manufacture of formulae for infants and young children are based on the concept that the products must be nutritionally adequate and microbiologically safe to consume. Thus, steps that eliminate or restrict microbiological growth are central to production processes. The processing technology for each specific formula is proprietary to the manufacturer but, in general, it involves the preservation of an oil-in-water (o/w) emulsion by dehydration in the case of powder products or, sterilization in the case of ready-to-feed or concentrated liquid products. Powdered infant formula may be produced using various processes, such as dry blending dehydrated ingredients to constitute a uniform formula or hydrating and wet-mixing a mixture of macro-ingredients, such as fat, protein and carbohydrate ingredients and then evaporating and spray drying the resultant mixture. A combination of the two processes described above may be used where a base powder is first produced by wet-mixing and spray drying all or some of the macro-ingredients and then dry blending the remaining ingredients, including carbohydrate, minerals and vitamins and other micronutrients, to create a final formula. Liquid formulae are available in a ready-to-feed format or as a concentrated liquid, which requires dilution, normally 1:1, with water. The manufacturing processes used for these products are similar to those used in the manufacture of recombined milk.

If it is desired to produce a liquid infant formula, the homogenized mixture is filled into suitable containers, preferably aseptically. However, the liquid composition may also be retorted in the container, suitable apparatus for carrying out the filling and retorting of this nature is commercially available.

The nutritional composition of the invention may also contain other substances which may have a beneficial effect, especially on bone health or bone development, such as lactoferrin, osteopontin, TGFbeta, slgA, glutamine, nucleotides, nucleosides, and the like.

Any reference to prior art documents in this specification is not to be considered an admission that such prior art is widely known or forms part of the common general knowledge in the field.

As used in this specification, the words "comprises", "comprising", and similar words, are not to be interpreted in an exclusive or exhaustive sense. In other words, they are intended to mean "including, but not limited to.

Unless defined otherwise, all technical and scientific terms used herein have the same meaning as commonly understood by one of ordinary skill in the art.

All percentages disclosed herein are on a w/w basis, unless stated otherwise.

### Example 1 :

Female Göttingen Minipigs (n=48, Ellegaard, Denmark) were randomly allocated to be artificially reared with milk substitutes containing blends of either sialylated human milk oligosaccharides (HMOs) (3'SL and 6'SL; 0.68 g/L, hereafter referred to as "2 HMOs blend"), N-acetylated and fucosylated HMOs (LNnT, LNT, 2'FL and di-FL; 4 g/L, hereafter referred to as "4 HMOs blend"), both sialylated HMOs, and N-acetylated and fucosylated HMOs(4 g/L, hereafter referred to as "6 HMOs blend") or lactose (4 g/L, herafter referred to as "MR") from 10 days to 11 weeks of age (weaning). The 2 HMOs and 4 HMOs blends are reference compositions. Starting at weaning, piglets were offered equal controlled amounts of a mild-western chow diet (13% protein, 14% fat, 33% cho, 4.4 MCals GE/kg) up to 48 weeks of age with ad libitum water. A naturally-reared reference group of piglets (NR; n=12) was kept with the sow until weaning and was fed the same diet afterwards. The minipigs were sacrificed at 12 months of age (adulthood equivalence). Tibia was directly excised and stored at -20C.

### 1) Measurement of bone mineral density (BMD)

BMD was measured by Dual energy X-ray absorptiometry (or DXA).

BMD corresponds to the bone mineral content divided by the surface of the tissue analysed and it is express in g/cm². Minipigs bones are in the range of human children bones sizes. We therefore use a DXA scanner with human parameters to evaluate the BMD of minipig tibias. Equipment used: Lunar iDXA (GE Healthcare) localized at VCLB metabolic unit and software: EnCORE 2011 (version 13.60.003, GE Healthcare). BMD have been analysed for the all tibia and different region of interest (proximal, midshaft and distal tibia) previously described in the literature (1).

### 2) Analysis of bone trabecular and cortical microstructure

Micro-computed tomography (µCT UCT40, Scanco Medical AG, Basserdorf Switzerland) was used to assess trabecular and cortical microstructure respectively investigated at proximal metaphysis and midshaft diaphysis tibia as previously described in the literature (2). Briefly, trabecular and cortical bone regions were evaluated using isotropic 12 µm voxels.

For the tibial trabecular region, to eliminate the primary spongiosa, 200 slices of primary spongiosa taken from the 100 slices of secondary spongiosa under the proximal growth plate were analysed. Tibial cortical structure was assessed using 50 continuous CT slides (600 µm) located at the tibial midshaft. Morphometric variables were computed from binarized images using direct, three-dimensional techniques that do not rely on prior assumptions about the underlying structure (3).

For the trabecular bone regions, the bone volume and tissue volume fraction (BV/TV), Trabecular Thickness (Tb.Th, µm), Trabecular Number (Tb.N, mm⁻¹) and trabecular connectivity density (Tb Conn Density, mm⁻³) were assessed.

For cortical bone at the femoral and tibial midshaft, the Cortical Tissue Volume (Ct.TV, mm³), Bone Volume (Ct.BV, mm³), the Marrow Volume (BMaV, mm³) and the average Cortical Thickness (Ct.Th, µm. were measured. In addition, cortical bone porosity (Ct.Po) in both metaphysis and diaphysis compartment was evaluated.

### 3) Results

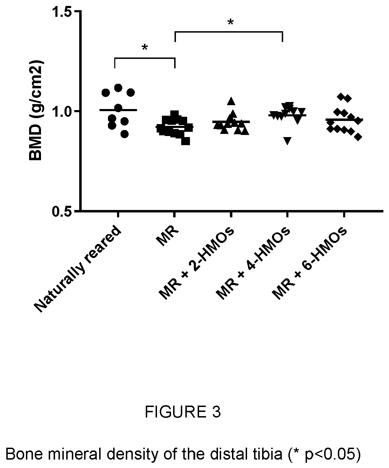

The results are shown in Figures 3-6, where the variations are measured with respect to the median, represented by the horizontal line.

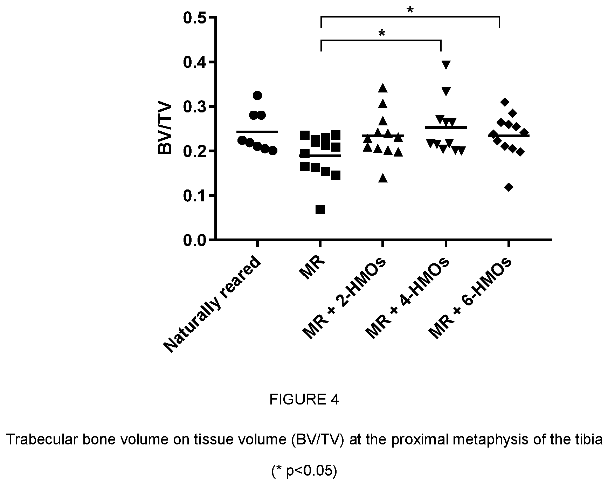

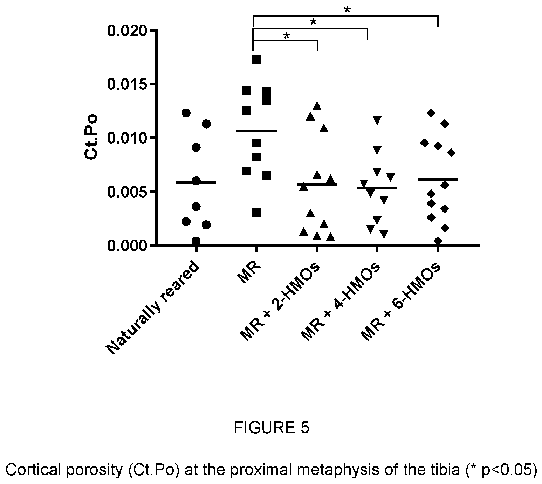

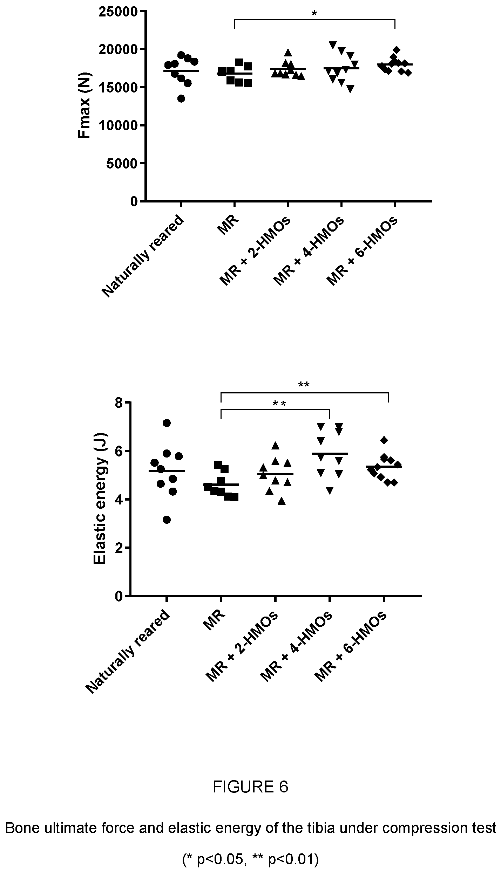

The results show that :
- A +6.2% increase in BMD was observed in the 4 HMOs blend compared to the MR-fed group (Figure 3).
- A +33.6% and +23.7% increase in BV/TV were observed in the 4 HMOs blend and 6 HMOs blend compared to the MR-fed group (Figure 4)
- A -46.7%, -50.1% and -42.6% decrease in Ct.Po were observed in the 2 HMOs, 4 HMOs and 6 HMOs blend compared to the MR-fed group (Figure 5)
- A +7.1% increase in ultimate load was observed in the 6 HMOs blend compared to the MR-fed group (Figure 6)
- A +27.5% and +16.3% increase in elastic energy were observed in the 4 HMOs blend and 6 HMOs blend compared to the MR-fed group (Figure 6)

### References

1. Bonnet N, Benhamou CL, Brunet-Imbault B, Arlettaz A, Horcajada MN, Richard O, et al. Severe bone alterations under beta 2 agonist treatment: bone mass, microarchitecture and strength analyses in female rats. Bone. 2005;37(5):622-33.
2. Bonnet N, Brun J, Rousseau JC, Duong LT, Ferrari SL. Cathepsin K Controls Cortical Bone Formation by Degrading Periostin. J Bone Miner Res. 2017;doi: 10:1002.
3. Bonnet N, Laroche N, Vico L, Dolleans E, Courteix D, Benhamou CL. Assessment of trabecular bone microarchitecture by two different x-ray microcomputed tomographs: a comparative study of the rat distal tibia using Skyscan and Scanco devices. Med Phys 2009;36(4):1286-97.

## Claims

1. A synthetic nutritional composition comprising a mixture of oligosaccharides, said mixture containing at least one sialylated oligosaccharide and at least one fucosylated oligosaccharide, for use in enhancing bone strength in a subject,
wherein the at least one sialylated oligosaccharide is a combination of 3'-sialyllactose (3'-SL) and 6'-sialyllactose (6'-SL),
wherein the at least one fucosylated oligosaccharide is a combination of 2'-fucosyllactose (2'FL) and 2',3-difucosyllactose (LDFT),
wherein the composition is further comprising at least one N-acetylated oligosaccharide,
wherein the at least one N-acetylated oligosaccharide is a combination of lacto-N-tetraose (LNT) and lacto-N-neotetraose (LNnT),
wherein said composition decreases cortical porosity (CtPo).

2. The synthetic nutritional composition for use according to claim 1, wherein the subject is a human child, infant or toddler.

3. A synthetic nutritional composition for use according to claim 1 or 2, wherein the oligosaccharide mixture comprises:
- 10 to 35 wt%, preferably 10 to 30 wt%, more preferably 10-25 wt%, with respect to the total weight of the oligosaccharide mixture, of at least one sialylated oligosaccharide;
- 30 to 80 wt%, preferably 40 to 80 wt%, more preferably 50 to70 wt%, with respect to the total weight of the oligosaccharide mixture, of at least one fucosylated oligosaccharide, and,
- 10 to 35 wt%, preferably 15 to 30 wt%, more preferably 15 to 20 wt%, of at least one N-acetylated oligosaccharide,
with respect to the total weight of the oligosaccharide mixture.

4. A synthetic nutritional composition for use according to any one of claims 1 to 3, where enhancement of bone strength comprises at least one of the following physiological processes: bone mass acquisition, optimization of peak bone mass, promotion of bone formation, promotion of bone anabolism, increase of bone mineral density and micro-architecture, modulation of bone biomechanical properties, modulation the ratio of bone formation and/or bone resorption, assist bone regeneration during fracture healing, regulation of bone resorption process.

5. A synthetic nutritional composition for use according to any one of claims 1 to 4, for further increasing bone mineral density (BMD), bone mineral content (BMC), bone volume and tissue volume fraction (BV/TV), bone ultimate force (FMax) and elastic energy.

6. A synthetic nutritional composition for use according to any one of claims 1 to 5, which is in the form of an infant formula.

7. A synthetic nutritional composition for use according to claim 6, wherein said infant formula is a preterm infant formula, a human milk fortifier, a starter infant formula, a follow-on formula, a baby-food formula, an infant cereal formula, a growing-up milk, a medical food product for clinical nutrition or a supplement.

8. A synthetic nutritional composition for use according to claim 1 or 2, wherein the composition comprises :
- 0.01 to 2wt%, preferably 0.05 to 1.5wt%, most preferably 0.07% to 1wt% of at least one sialylated oligosaccharide,
- 0.05 to 3wt%, preferably 0.1 to 2wt%, most preferably 0.2 to 1.5wt% of at least one fucosylated oligosaccharide, and,
- 0.01 to 1wt%, preferably 0.03 to 0.6wt%, most preferably 0.05 to 0.5wt% of at least one N-acetylated oligosaccharide,
with respect to the total weight of the infant formula under powder form.

## Patentansprüche

1. Synthetische Ernährungszusammensetzung, umfassend eine Mischung von Oligosacchariden, wobei die Mischung mindestens ein sialyliertes Oligosaccharid und mindestens ein fucosyliertes Oligosaccharid enthält, zur Verwendung bei der Verbesserung der Knochenfestigkeit in einem Subjekt,
wobei das mindestens eine sialylierte Oligosaccharid eine Kombination aus 3'-Sialyllactose (3'-SL) und 6'-Sialyllactose (6'-SL) ist,
wobei das mindestens eine fucosylierte Oligosaccharid eine Kombination aus 2'-Fucosyllactose (2'FL) und 2',3-Difucosyllactose (LDFT) ist,
wobei die Zusammensetzung ferner mindestens ein N-acetyliertes Oligosaccharid umfasst,
wobei das mindestens eine N-acetylierte Oligosaccharid eine Kombination aus Lacto-N-tetraose (LNT) und Lacto-N-neotetraose (LNnT) ist,
wobei die Zusammensetzung die kortikale Porosität (CtPo) verringert.

2. Synthetische Ernährungszusammensetzung zur Verwendung nach Anspruch 1, wobei das Subjekt ein menschliches Kind, ein Säugling oder ein Kleinkind ist.

3. Synthetische Ernährungszusammensetzung zur Verwendung nach Anspruch 1 oder 2, wobei die Oligosaccharidmischung umfasst:
- 10 bis 35 Gew.-%, vorzugsweise 10 bis 30 Gew.-%, mehr bevorzugt 10 bis 25 Gew.-% mindestens eines sialylierten Oligosaccharids, bezogen auf das Gesamtgewicht der Oligosaccharidmischung;
- 30 bis 80 Gew.-%, vorzugsweise 40 bis 80 Gew.-%, mehr bevorzugt 50 bis 70 Gew.-% mindestens eines fucosylierten Oligosaccharids, bezogen auf das Gesamtgewicht der Oligosaccharidmischung und,
- 10 bis 35 Gew.-%, vorzugsweise 15 bis 30 Gew.-%, mehr bevorzugt 15 bis 20 Gew.-% mindestens eines N-acetylierten Oligosaccharids,
bezogen auf das Gesamtgewicht der Oligosaccharidmischung.

4. Synthetische Ernährungszusammensetzung zur Verwendung nach einem der Ansprüche 1 bis 3, wobei die Verbesserung der Knochenfestigkeit mindestens einen der folgenden physiologischen Prozesse umfasst: Aufbau von Knochenmasse, Optimierung von maximaler Knochenmasse, Förderung von Knochenbildung, Förderung von Knochenanabolismus, Erhöhung von Knochenmineraldichte und Mikroarchitektur, Modulation von biomechanischen Eigenschaften des Knochens, Modulation des Verhältnisses von Knochenbildung und/oder Knochenresorption, Unterstützung der Knochenregeneration während der Frakturheilung, Regulation des Knochenresorptionsprozesses.

5. Synthetische Ernährungszusammensetzung zur Verwendung nach einem der Ansprüche 1 bis 4 zum weiteren Erhöhen der Knochenmineraldichte (BMD), des Knochenmineralgehalts (BMC), des Knochenvolumens und der Gewebevolumenfraktion (BV/TV), der maximalen Knochenkraft (FMax) und der elastischen Energie.

6. Synthetische Ernährungszusammensetzung zur Verwendung nach einem der Ansprüche 1 bis 5, die in Form einer Säuglingsnahrung vorliegt.

7. Synthetische Ernährungszusammensetzung zur Verwendung nach Anspruch 6, wobei die Säuglingsnahrung eine Frühgeborensäuglingsnahrung, ein Muttermilchstärkungsmittel, eine Starter-Säuglingsnahrung, eine Folgenahrung, eine Babynahrung, eine Säuglingsgetreidenahrung, eine Wachstumsmilch, ein medizinisches Nahrungsprodukt für die klinische Ernährung oder ein Ergänzungsmittel ist.

8. Synthetische Ernährungszusammensetzung zur Verwendung nach Anspruch 1 oder 2, wobei die Zusammensetzung umfasst:
- 0,01 bis 2 Gew.-%, vorzugsweise 0,05 bis 1,5 Gew.-%, am meisten bevorzugt 0,07 % bis 1 Gew.-% mindestens eines sialylierten Oligosaccharids,
- 0,05 bis 3 Gew.-%, vorzugsweise 0,1 bis 2 Gew.-%, am meisten bevorzugt 0,2 bis 1,5 Gew.-% mindestens eines fucosylierten Oligosaccharids und,
- zu 0,01 bis 1 Gew.-%, vorzugsweise 0,03 bis 0,6 Gew.-%, am meisten bevorzugt 0,05 bis 0,5 Gew.-% mindestens eines N-acetylierten Oligosaccharids,
bezogen auf das Gesamtgewicht der Säuglingsnahrung in Pulverform.

## Revendications

1. Composition nutritionnelle synthétique comprenant un mélange d'oligosaccharides, ledit mélange contenant au moins un oligosaccharide sialylé et au moins un oligosaccharide fucosylé, pour une utilisation dans l'amélioration de la résistance osseuse chez un sujet,
dans laquelle l'au moins un oligosaccharide sialylé est une combinaison de 3'-sialyllactose (3'-SL) et de 6'-sialyllactose (6'-SL),
dans laquelle l'au moins un oligosaccharide fucosylé est une combinaison de 2'-fucosyllactose (2'FL) et de 2',3-difucosyllactose (LDFT),
dans laquelle la composition comprend en outre au moins un oligosaccharide N-acétylé,
dans laquelle l'au moins un oligosaccharide N-acétylé est une combinaison de lacto-N-tétraose (LNT) et de lacto-N-néotétraose (LNnT),
dans laquelle ladite composition diminue la porosité corticale (CtPo).

2. Composition nutritionnelle synthétique pour une utilisation selon la revendication 1, dans laquelle le sujet est un enfant, un nourrisson ou un enfant en bas âge.

3. Composition nutritionnelle synthétique pour une utilisation selon la revendication 1 ou 2, dans laquelle le mélange d'oligosaccharides comprend :
• de 10 à 35 % en poids, de préférence de 10 à 30 % en poids, plus préférablement de 10 à 25 % en poids, par rapport au poids total du mélange d'oligosaccharides, d'au moins un oligosaccharide sialylé ;
- de 30 à 80 % en poids, de préférence de 40 à 80 % en poids, plus préférablement de 50 à 70 % en poids, par rapport au poids total du mélange d'oligosaccharides, d'au moins un oligosaccharide fucosylé, et
- de 10 à 35 % en poids, de préférence de 15 à 30 % en poids, plus préférablement de 15 à 20 % en poids, d'au moins un oligosaccharide N-acétylé, par rapport au poids total du mélange d'oligosaccharides.

4. Composition nutritionnelle synthétique pour une utilisation selon l'une quelconque des revendications 1 à 3, dans laquelle l'amélioration de la résistance osseuse comprend au moins l'un des processus physiologiques suivants : l'acquisition de masse osseuse, l'optimisation de la masse osseuse maximale, la promotion de la formation osseuse, la promotion de l'anabolisme osseux, l'augmentation de la densité minérale osseuse et de la microarchitecture, la modulation des propriétés biomécaniques osseuses, la modulation du rapport de formation osseuse et/ou de résorption osseuse, l'aide à la régénération osseuse pendant la guérison des fractures, la régulation du processus de résorption osseuse.

5. Composition nutritionnelle synthétique pour une utilisation selon l'une quelconque des revendications 1 à 4, pour augmenter en outre la densité minérale osseuse (BMD), le contenu minéral osseux (BMC), la fraction du volume osseux et du volume tissulaire (BV/TV), la force ultime osseuse (FMax) et l'énergie élastique.

6. Composition nutritionnelle synthétique pour une utilisation selon l'une quelconque des revendications 1 à 5, laquelle se présente sous la forme d'une préparation pour nourrissons.

7. Composition nutritionnelle synthétique pour une utilisation selon la revendication 6, dans laquelle ladite préparation pour nourrissons est une préparation pour nourrissons prématurés, un fortifiant pour lait maternel, une préparation pour nourrissons de premier âge, une préparation de suite, une préparation d'aliments pour bébés, une préparation de céréales pour nourrissons, un lait de croissance, un produit alimentaire médical pour la nutrition clinique ou un supplément.

8. Composition nutritionnelle synthétique pour une utilisation selon la revendication 1 ou 2, dans laquelle la composition comprend :
- de 0,01 à 2 % en poids, de préférence de 0,05 à 1,5 % en poids, le plus préférablement de 0,07 % à 1 % en poids d'au moins un oligosaccharide sialylé,
- de 0,05 à 3 % en poids, de préférence de 0,1 à 2 % en poids, le plus préférablement de 0,2 à 1,5 % en poids d'au moins un oligosaccharide fucosylé, et
- de 0,01 à 1 % en poids, de préférence de 0,03 à 0,6 % en poids, le plus préférablement de 0,05 à 0,5 % en poids d'au moins un oligosaccharide N-acétylé,
par rapport au poids total de la préparation pour nourrissons sous forme de poudre.
